Europäisches Patentamt

European Patent Office

Office européen des brevets

(11) Veröffentlichungsnummer: **0 196 554 B1**

(12) **EUROPÄISCHE PATENTSCHRIFT**

(45) Veröffentlichungstag der Patentschrift: 23.05.90

(21) Anmeldenummer: 86103758.8

(22) Anmeldetag: 19.03.86

(51) Int. Cl.⁵: **C 07 C 255/04, C 07 C 253/22**

(54) Verfahren zur Herstellung von aliphatischen Dinitrilen.

(30) Priorität: 26.03.85 DE 3510876

(43) Veröffentlichungstag der Anmeldung:
08.10.86 Patentblatt 86/41

(45) Bekanntmachung des Hinweises auf die
Patenterteilung:
23.05.90 Patentblatt 90/21

(84) Benannte Vertragsstaaten:
BE DE FR GB IT NL

(56) Entgegenhaltungen:
DE-B-1 188 578
FR-A-2 069 574
GB-A-1 397 729

CHEMICAL ABSTRACTS, Band 84, Nr. 1, 5.
Januar 1976, Seite 379, Nr. 4433a, Columbus,
Ohio, US; COSTA NOVELLA, E.: "Nitrilation of
pelargonic acid"

(73) Patentinhaber: BASF Aktiengesellschaft
Carl-Bosch-Strasse 38
D-6700 Ludwigshafen (DE)

(72) Erfinder: Hoelderich, Wolfgang, Dr.
Mannheimer Strasse 18 c
D-6710 Frankenthal (DE)
Erfinder: Lendle, Hubert, Dr.
Ruedigerstrasse 41
D-6700 Ludwigshafen (DE)
Erfinder: Magnussen, Peter, Dr.
Professor-Dillinger-Weg 25
D-6702 Bad Duerkheim (DE)
Erfinder: Leitner, Hans, Dr.
Taunusstrasse 22
D-6710 Frankenthal (DE)
Erfinder: Manegold, Jost Henrich, Dr.
Neustadter Strasse 29
D-6715 Lambsheim (DE)
Erfinder: Leitenberger, Wolfgang, Dr.
Ludwig-Beck-Strasse 47
D-6800 Mannheim (DE)

Courier Press, Leamington Spa, England.

**Beschreibung**

Ein in der Technik vielfach verwendetes Verfahren zur Herstellung von aliphatischen Dinitrilen ist die Umsetzung von aliphatischen Dicarbonsäuren mit Ammoniak in Gegenwart von Katalysatoren, z.B. in einem fluidisierten Katalysatorbett. Als Katalysatoren eignen sich wasserabspaltende Mittel, wie Kieselgel. Borphosphat, Tonerde oder Mischungen derselben, die durch geringe Mengen an Alkalioxid und Zusatz saurer Stoffe, wie Phosphorsäure, Borsäure, Vanadinsäure, Molybdänsäure, Wolframsäure oder andere Heteropolysäuren aktiviert sein könne, wie aus der DE—OS 11 88 578 und DE—OS 20 56 295 bekannt ist. Die bekannten Verfahren haben den Nachteil, daß die Katalysatoren schnell desaktivieren und ihre Selektivität einbüßen. Aufgrund der häufig notwendigen Katalysatorregenrierung bei hohen Temperaturen rekristallisiert z.B. amorphes Siliciumdioxid zu Cristobalit. Dies führt zu einer irreversiblen Schädigung des Katalysators. Diese Nachteile gelten auch für Katalysatoren, in den Phosphorsäure in einem chemischen Unterschuß gegenüber dem Alkaligehalt des Siliciumdioxidträgers vorliegen, wie sie aus der DE—OS 20 56 295 bekannt sind.

Es war deshalb die technische Aufgabe gestellt, ein Verfahren zur Herstellung von aliphatischen Dinitrilen durch Umsetzung von aliphatischen Dicarbonsäuren und Ammoniak zur Verfügung zu stellen, bei dem die mitverwendeten Katalysatoren eine hohe Selektivität und lange Lebensdauer aufweisen und leicht ohne Verlust an Aktivität und Selektivität regeneriert werden können und wenig Nebenprodukte gebildet werden.

Diese Aufgabe wird gelöst in einem Verfahren zur Herstellung von aliphatischen Dinitrilen durch Umsetzung von aliphatischen Dicarbonsäuren mit Ammoniak im Überschuß bei einer Temperatur von 200 bis 500°C in Gegenwart von Katalysatoren, durch gekennzeichnet, daß man Zeolithe als Katalysatoren verwendet.

Das neue Verfahren hat den Vorteil, daß sich die Katalysatoren leicht regenerieren lassen und auch nach mehrmaligem Regenerieren eine hohe Katalysatoreaktivität und Selektivität aufweisen. Ferner hat das neue Verfahren den Vorteil, daß man eine sehr hohe Selektivität erzielt.

Bevorzugte aliphatische Dicarbonsäuren als Ausgangsstoffe sind solche der Formel (I) HOOC—R—COOH, in der R einen aliphatischen Rest mit 2 bis 12 Kohlenstoffatomen bezeichnet. In bevorzugten Ausgangsstoffen der Formel I bezeichnet R einen Alkylenrest mit 2 bis 12, insbesondere mit 2 bis 8 Kohlenstoffatomen. Geeignete Dicarbonsäuren sind beispielsweise Bernsteinsäure, Glutarsäure, α-Methylglutarsäure, Adipinsäure, Azelainsäure oder Sebacinsäure. Besondere technische Bedeutung hat Adipinsäure erlangt.

Die Umsetzung erfolgt mit überschüssigem Ammoniak. Verteilhaft wenden man je Mol Dicarbonsäure 4 bis 20, insbesondere 6 bis 12 Mol Ammoniak an.

Die Umsetzung kann in der Gasphase oder in der flüssigen Phasen, z.B. unter Mitverwendung von Verdünnungsmitteln wie dem jeweils erzeugten Dinitril durchgeführt werden. Vorteilhaft führt man die Umsetzung in der Gasphase durch.

Für die Umsetzung wendet man im allgemeinen Temperaturen von 200 bis 500°C an. Falls man die Umsetzung in flüssiger Phase durchführt, haben sich Temperaturen von 250 bis 350°C, insbesondere 280 bis 330°C, bewährt. Wird die Umsetzung in der Gasphase durchgeführt, so wendet man vorteilhaft Temperaturen von 300 bis 500°C, insbesondere 350 bis 420°C, an.

Erfindungsgemäß verwendet man als Katalysatoren Zeolithe, Zeolithe sind kristalline Aluminosilikate, die eine hochgeordnete Struktur mit einem starren dreidimensionalen Netzwerk von $SiO_4$- und $AlO_4$-Tetraedern bisitzen, die durch gemeinsame Sauerstoffatome verbunden sind. Das Verhältnis von Silicium- und Aluminiumatomen zu Sauerstoff beträgt 1:2. Die Elektrovalenz der Aluminium enthaltenden Tetraeder ist durch Einschluß von Kationen in den Kristall, z.B. eines Alkali- oder Wasserstoffatoms, ausgeglichen. Ein Kationenaustausch ist möglich. Die Räume zwischen den Tetraedern sind vor der Dehydration durch Trocknen bzw. Calcinieren von Wassermolekülen besetzt. Die Zeolithe können auch anstelle des Aluminiums dreiwertige Elemente, wie Bor, Gallium, Eisen oder Chrom und anstelle des Siliciums vierwertige Elemente wie Germanium, Titan, Zirkonium oder Hafnium enthalten.

Vorzugsweise werden als Katalysatoren Zeolithe des Pentasiltyps eingesetzt. Diese Zeolithe können unterschiedliche chemische Zusammensetzungen aufweisen. Es handelt sich hierbei um Alumino-, Boro-, Eisen-, Gallium-, Chrom, Arsen- oder Wismutsilikatzeolithe oder deren Gemische sowie um Alumino-, Boro-, Gallium- oder Eisengermanatzeolithe oder deren Gemische. Besonders bevorzugt sind Boro- und Eisensilikatzeolithe des Pentasiltyps.

Der Borosilikatzeolith wird z.B. bei 90 bis 200°C unter autogenem Druck hergestellt, indem man eine Borverbindung, wie Borsäure mit einer Siliciumverbindung, vorzugsweise mit hochdispersem Siliciumdioxid in wäßriger Aminlösung, insbesondere in 1,6-Hexandiamin- oder 1,3-Propandiamin- oder Triethylentetramin-Lösung mit oder ohne Alkali- oder Erdalkalizusatz umsetzt. Anstelle einer wäßrigen Aminlösung kann auch eine etherische Lösung, z.B. Diethylenglykoldimethylether, oder eine alkoholische Lösung, z.B. 1,6-Hexandiol, verwendet werden.

Den Eisensilikatzeolith erhält man z.B. aus einem Eisen-III-Salz, vorzugsweise Eisen-III-Sulfat und einer Siliciumverbindung, vorzugsweise hochdispersem Siliciumdioxid, in wäßriger Aminlösung insbesondere 1,6-Hexandiamin mit und ohne Alkali- oder Erdalkalizusatz bei 100 bis 200°C unter autogenem Druck.

Die so hergestellten Boro- und Eisensilikatzeolithe werden vorteilhaft unmittelbar nach ihrer Isolierung und Tocknung bei 100 bis 160°C, vorzugsweise 110°C, und Calcination bei 450 bis 550°C, vorzugsweise 500 bis 540°C, zu Strängen oder Wirbelgut verarbeitet. Die Verformung kann z.B. auch mit einem Bindemittel im Verhältnis 95:5 bis 30:70 vorgenommen werden. Als Bindemittel eignen sich Siliciumdioxid, bevorzugt Kieselgel, Kieselsol oder hochdisperses Siliciumdioxid, hochdisperses Titandioxid, amorphe Aluminosilikate mit einem Verhältnis von Siliciumdioxid und Aluminiumoxid wie 25:70 bis 95:5 sowie Aluminiumoxide.

Nach der Verformung werden die Extrudate, die Preßlinge oder das Wirbelgut z.B. bei 110°C 16 Stunden getrocknet und bei 500°C 16 Stunden calciniert. Besonders vorteilhaft lassen sich solche Katalysatoren herstellen, indem man den isolierten Boro- oder Eisensilikatzeolith direkt nach der Trocknung verformt und erstmals nach der Vorformung calciniert. Aus den zu Strängen verformten Katalysatoren kann man z.B. durch Mahlen und Sieben Wirbelgut mit einer Teilchengröße von 0.05 bis 0,5 mm erhalten.

Liegt der Zeolith aufgrund der Art seiner Herstellung nicht in der katalytisch bevorzugten aciden H-Form vor, sondern z.B. in der Na-Form, dann kann diese durch Ionenaustausch mit Ammoniumionen und anschließende Calcination oder durch Behandlung mit Säuren vollkommen oder partiell in die gewünschte H-Form überführt werden. Man kann an den Zeolithen zur Erhöhung der Selektivität, der Standzeit und der Anzahl der Regenierungen auch unterschiedliche Modifizierungen vonehmen. Eine geeignete Modifizierung besteht z.B. darin, daß man den unverformten oder verformten Zeolithen mit Alkalimetallen wie Natriumnitrat- sofern nicht schon von der Synthese her die Alkali-Form des Zeolithen vorliegt -mit Erdalkali wie Calcium, Magnesium, mit Erdmetallen wie Bor, Tallium, mit Übergangsmetallen wie Molybdän, Wolfram, Eisen, Zink, Kupfer, mit Edelmetallen wie Palladium oder seltenen Erden wie Cer oder Lanthan in Form deren Salze ionentauschen bzw. imprägnieren kann.

Vorteilhaft stellt mach solche modifizierten Zeolithe her, indem man die verformten Pentasilzeolithe in einem Steigrohr vorlegt und bei 20 bis 100°C z.B. eine wäßrige Lösung eines Halogenids oder Nitrats der voranbeschriebenen Metalle darüberleitet. Ein derartiger Ionentausch kann z.B. an der Wasserstoff-, Ammonium- und Alkaliform des Zeolithen vorgenommen werden. Man kann die Metallaufbringung auf den Zeolithen z.B. auch so vornehmen, daß man das zeolithische Material z.B. mit einem Halogenid, einem Nitrat oder einem Oxid der voranbeschreibenen Metalle in wäßriger oder alkoholischer Lösung imprägniert. Sowohl an einem Ionenaustausch als auch an eine Imprägnierung schließt sich zumindestens eine Trocknung und wahlweise eine abermalige Calcination an.

Bei Zeolithen die mit Metallen wie Palladium oder Platin dotiert sind, ist eine Nachbehandlung mit Wasserstoff vorteilhaft.

Eine weitere Möglichkeit der Modifizierung besteht darin, daß man die Zeolithe—vorformt oder unverformt—einer Behandlung mit Säuren wie Phosphorsäure, Salzsäure oder Flußsäure und/oder mit Wasserdampf unterwirft. Dabei geht man vorteilhaft z.B. so vor, daß man Zeolithe in Pulverform mit 1 n Phosphorsäure 1 Stunde bei 80°C behandelt. Nach der Behandlung wird mit Wasser gewaschen, bei 110°C 16 Stunden getrocknet und bei 500°C 20 Stunden calciniert. Nach einer anderen Arbeitsweise behandelt man Zeolithe vor oder nach ihrer Verformung mit Bindemitteln z.B 1 bis 3 Stunden bei Temperaturen von 60 bis 80°C mit einer 3- bis 25 gew.%igen, insbesondere 12- bis 20 gew.%igen wäßrigen Salzsäure. Anschließend wird der so behandelte Zeolith mit Wasser gewaschen, bei 100 bis 160°C getrocknet und bei 400 bis 500°C calciniert.

Nach einer anderen Arbeitsweise lassen sich Zeolithe durch Aufbringung von Phosphorverbindungen, wie Trimethoxyphosphat, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger $NaH_2PO_4$-Lösung getränkt bei 110°C getrocknet und bei 500°C calciniert.

Nach einer Desaktivierung der Zeolithkatalysatoren, die bei dem Verfahren nach der Erfindung durch Koksabscheidung eintreten kann, lassen sich die Katalysatoren durch Abbrennen der Koksablagerung mit Luft oder mit einem Gemisch aus Luft und Stickstoff bei 400 bis 550°C, vorzugsweise 500 bis 540°C in einfacher Weise regenerieren, wodurch sie ihre Anfangsaktivität zurückerhalten. Man kann auch durch eine partielle Verkokung die Aktivität des Katalysators für ein Selektivitätsoptimum des gewünschten Reaktionsproduktes einstellen. Wird die Umsetzung der Dicarbonsäure mit Ammoniak unter Mitverwendung von Gasen wie Wasserstoff, Stickstoff oder Wasserdampf durchgeführt, so kann damit die Produktzusammensetzung sowie die Standzeit des Katalysators beeinflußt werden.

Im allgemeinen werden die Katalysatoren wahlweise als Stränge von 2 bis 4 mm Länge, als Tabletten mit 3 bis 5 mm Durchmesser oder als Wirbelgut mit einer Teilchengröße von 0,05 bis 0,5 mm verwendet. Das Wirbelgut läßt sich durch Zerkleinern und Aussieben von Strängen oder durch Sprühtrocknung herstellen.

Vorteilhaft wird die Umsetzung in einem auf- und abwirbelnden Katalysatorbett durchgeführt, wobei man von unten gasförmigen Ammoniak einleitet und verdampfte Dicarbonsäure zuführt oder diese im Wirbelbett verdampft. Andererseits kann die Umsetzung auch an einem fest angeordneten Katalysator oder in flüssiger Phase mit suspendiertem Katalysator durchgeführt werden.

Nach dem Verfahren der Erfindung hergestellte Dinitrile eignen sich zur Herstellung von Diaminen, die Ausgangsstoffe für Polyamide sind.

Das Verfahren nach der Erfindung sei an folgenden Beispielen veranschaulicht.

EP 0 196 554 B1

Der in den Beispielen verwendete Katalysator A wird wie folgt hergestellt:

## Katalysator A

Der Zeolith wurde in einer hydrothermalen Synthese aus 640 g $SiO_2$ (hochdisperse Kieselsäure), 122 g $H_3BO_3$, 800 g einer wäßrigen Hexandiamin-Lösung (Mischung 50:50 Gew.%) bei 170°C unter autogenem Druck in einem Rührautoklaven hergestellt. Nach Abfiltrieren und Auswaschen wurde das kristalline Reaktionsprodukt bei 100°C/24 h getrocknet und bei 500°C/24 calciniert. Es wurde ein Borosilikatzeolith vom Pentasiltyp erhalten, der 94,2 Gew.% $SiO_2$ und 2,3 Gew.% $B_2O_3$ enthielt. Dieses Zeolithpulver wird mit Kieselgel im Gewichtsverhältnis 90:10 zu 5 mm Strängen verformt und bei 110°C/16 h getrocknet. Diese Stränge werden zu einem Wirbelgut mit einer Siebfraktion von 50 bis 500 µ zerkleinert. Danach schließt sich eine Calcinierung bei 500°C/16 h an. 600 g dieses Wirbelgutes werden mit 283 g $NaH_2PO_4 \cdot H_2O$— in 500 g $H_2O$ gelöst-getränkt, danach bei 110°C getrocknet und bei 500°C/14 h calciniert. Der Katalysator A enthält 7,6 Gew.% P und 5,6 Gew.% Na.

## Beispiel I

500 g des Katalysators A der Kornfraktion mit Durchmessern zwischen 0,06 mm und 0,3 mm werden in einem Wirbelbettreaktor mit einem Innendurchmesser von 60 mm eingebaut und in einem Fluidisierungsstrom von 300 Nl/h Ammoniak auf 350°C erhitzt. Die geschuppte Adipinsäure (1000 g) wird über eine Zellradschleuse mit einer Rate von 200 g/h in einen 100 Nl/h betragenden Fördergasstrom ($NH_3$) eindosiert und in das untere Viertel des Wirbelbetts befördert. Die Umsetzungsprodukte werden aus dem Reaktionsgas durch Kondensation auf Gastemperaturen von ca. 0°C gewonnen. Die sich bildenden Phasen werden durch Strippen mit $N_2$ von gelöstem Ammoniak befreit, voneinander getrennt, gewogen und deren Gehalt durch GC-Analyse bestimmt. Es wurden folgende Ausbeuten erzielt:

| | |
|---|---|
| Adiponitril | 87,4% (mol/mol) |
| Cyanvaleriansäure | 1,5% (mol/mol) |
| Cyanvalerianamid | 7,1% (mol/mol) |
| Σ Wertprodukte | 96,0% (mol/mol) |
| Cyancyclopentanonimin | 1,1% (mol/mol) |
| Cyclopentanon | 1,0% (mol/mol) |
| | 98,1% (mol/mol) |

## Beispiel II

Man verfährt in gleicher Weise wie bei Beispiel I, wendet jedoch eine Temperatur von 400°C an, hierbei erhält man:

| | |
|---|---|
| Adiponitril | 94 % (mol/mol) |
| Cyanvalerinsäure | 0,7% (mol/mol) |
| Cyanvalerianamid | 2,8% (mol/mol) |
| Σ Wertprodukte | 97,5% (mol/mol) |
| Cyancyclopentanonimin | 0,5% (mol/mol) |
| Cyclopentanon | 0,9% (mol/mol) |
| | 98,9% (mol/mol) |

## Beispiel III

Man verfährt in gleicher Weise wie bei Beispiel I, wendet jedoch eine Temperatur von 420°C an, hierbei erhält man:

| | |
|---|---|
| Adiponitril | 94,8% (mol/mol) |
| Cyanvaleriansäure | 0,4% (mol/mol) |
| Cyanvalerianamid | 1,2% (mol/mol) |
| Σ Wertprodukte | 96,4% (mol/mol) |
| Cyancyclopentanonimin | 1,1% (mol/mol) |
| Cyclopentanon | 0,8% (mol/mol) |
| | 98,3% (mol/mol) |

4

Katalysator B

Das bei Katalysator A beschreibene Wirbelgut wird nunmehr mit $O=P(OCH_3)_3$ (Trimethoxyphosphat) anstatt Natriumdihydrogenphosphat dotiert. 600 g des Wirbelgutes werden mit einer Mischung aus 287 g $O=P(OCH_3)_3$ und 360 g $H_2O$ getränkt, danach bei 150°C getrocknet und bei 500°C/14 h calciniert. Der Phosphorgehalt des Katalysators B beträgt 2,16%.

Beispiel IV

Man verfährt wie in Beispiel I beschrieben, führt die Umsetzung jedoch bei 410°C unter Verwendung des Katalysators B anstatt A durch. Man erhält folgende Ergebnisse:

| | |
|---|---|
| Adiponitril | 86.2% (mol/mol) |
| Cyanvaleriansäure | 0,4% (mol/mol) |
| Cyanvalerianamid | 2,0% (mol/mol) |
| Σ Wertprodukte | 88,6% (mol/mol) |
| Cyancyclopentanonimin | 2,4% (mol/mol) |
| Cyclopentanon | 3,2% (mol/mol) |
| | 94,2% (mol/mol) |

Vergleichsbeispiel

Man verfährt wie in Beispiel I beschrieben, verwendet jedoch $SiO_2$ als Katalysator und hält eine Temperatur von 480°C ein. Hierbei erzielt man folgende Ergebnisse:

| | |
|---|---|
| Adiponitril | 83,4% (mol/mol) |
| Cyanvaleriansäure | 3,0% (mol/mol) |
| Cyanvalerianamid | 0,6% (mol/mol) |
| Σ Wertprodukte | 87,0% (mol/mol) |
| Cyancyclopentanonimin | 2,4% (mol/mol) |
| Cyclopentanon | 4,3% (mol/mol) |

**Patentansprüche**

1. Verfahren zur Herstellung von aliphatischen Dinitrilen durch Umsetzung von aliphatischen Dicarbonsäuren mit Ammoniak im Überschuß bei einer Temperatur von 200 bis 500°C in Gegenwart von Katalysatoren, dadurch gekennzeichnet, daß man Zeolithe als Katalysatoren verwendet.

2. Verfahren nach Anspruch 1, dadurch gekennzeichnet, daß man Zeolithe des Pentasiltyps verwendet.

3. Verfahren nach den Ansprüchen 1 bis 2, dadurch gekennzeichnet, daß man Borosilikatzeolithe des Pentasiltyps verwendet.

4. Verfahren nach den Ansprüchen 1 und 2, dadurch gekennzeichnet, daß man Eisensilikatszeolithe des Pentasiltyps verwendet.

5. Verfahren nach den Ansprüchen 1 bis 4, dadurch gekennzeichnet, daß die Zeolithe mit Phosphorverbindungen dotiert sind.

6. Verfahren nach den Ansprüchen 1 bis 5, dadurch gekennzeichnet, daß die Zeolithe mit Alkaliionen dotiert sind.

7. Verfahren nach den Ansprüchen 1 bis 6, dadurch gekennzeichnet, daß die Umsetzung im Wirbelbett durchgeführt wird.

**Revendications**

1. Procédé de préparation de dintriles aliphatiques par réaction de diacides carboxyliques aliphatiques avec de l'ammoniac en excès à une température de 200 à 500°C en présence de catalyseurs, caractérisé en ce qu'on utilise une zéolithe comme catalyseur.

2. Procédé selon la revendication 1, caractérisé en ce qu'on utilise une zéolithe de type pentasil.

3. Procédé selon les revendications 1 et 2, caractérisé en ce qu'on utilise une zéolithe de borosilicate de type pentasil.

4. Procédé selon les revendications 1 et 2, caractérisé en ce qu'on utilise une zéolithe de ferrosilicate de type pentasil.

5. Procédé selon les revendications 1 à 4, caractérisé en ce que les zéolithes sont dopées avec des composés du phosphore.

6. Procédé selon les revendications 1 à 5, caractérisé en ce que les zéolithes sont dopées avec des ions alcalins.

7. Procédé selon les revendications 1 à 6, caractérisé en ce qu'on mène la réaction en lit fluidisé.

**Claims**

1. A process for preparing aliphatic dinitriles, by reacting aliphatic dicarboxylic acids with ammonia in excess at from 200 to 500°C in the presence of catalysts, wherein the catalysts used are zeolites.

2. A process as claimed in Claim 1, wherein zeolites of the pentasil type are used.

3. A process as claimed in Claims 1 and 2, wherein borosilicate zeolites of the pentasil type are used.

4. A process as claimed in Claims 1 and 2, wherein iron silicate zeolites of the pentasil type are used.

5. A process as claimed in Claims 1 to 4, wherein the zeolites are doped with phosphorus compounds.

6. A process as claimed in Claims 1 to 5, wherein the zeolites are doped with alkali metal ions.

7. A process as claimed in Claims 1 to 6, wherein the reaction is carried out in a fluidized bed.